# EUROPEAN PATENT SPECIFICATION

(11) **EP 1 695 678 B1**
(45) Date of publication and mention of the grant of the patent: **08.08.2007**
(21) Application number: 05075477.9
(22) Date of filing: 28.02.2005
(51) Int. Cl.: A61F 5/44

(54) **Disposable fluid collection bag for hygienic purposes**
Hygienischer Einwegsammelbeutel für hygienische Anwendung
Sac collecteur hygienic et jetable de fluides biologiques

(43) Date of publication of application: 30.08.2006
(73) Proprietor: Unomedical A/S, 3460 Birkerød (DK)
(72) Inventor: Nordbo, Kalle Jon, 2300 Copenhagen S (DK); Göransson, Magnus, 214 21 Malmö (SE); Hansen, Trygve Kalf, 4040 Jyllinge (DK)
(74) Representative: Elmeros, Claus

(56) References cited:
- EP-A- 0 847 742
- EP-A- 1 473 001
- GB-A- 2 336 830
- US-A- 4 990 145
- US-A- 5 569 225

## Description

The present invention relates to a disposable fluid collection bag, in particular a urine bag of the kind mentioned in the preamble of claim 1.

Document EP 0 847 742 is considered to represent the closest prior art.

From EP 0 748 620 B1 and EP 0 847 742 B1, a fluid collection bag for use for hygienic purposes of this kind is known. This type of disposable urine bag disclosed in EP 0 748 620 B and EP 0 847 742 B1 comprises a collar having an opening into a plastic pouch having two walls. Between said walls is provided a non-return valve is integrally formed. The non-return flow arrangement consists of three pairs of plastic foil sheets extending down from the opening with increasing lengths, loosely held in place by point welding spots on each pair of foil sheets transversely of the pouch in order to divide the pouch into an upper and lower part. The upper part is provided with a weld along the edge of the pouch to seal it.

A disadvantage of this urine bag is the limitations in relation to its manufacturing, in particular to the welding of the urinal. It is found complicated to join together eight layers by welding in a fluid tight manner since the welding must join together two pouch walls and six valve layers, i.e. three pairs for forming a non-return flow system below the inlet. The side walls are typically made of a first type of plastic material, whereas the non-return flow layers are of another and/or individually different plastic material. This results in different welding conditions along the bag as the number of layers and the types of materials differ depending on which part of the bag is to be welded. This incurs extra production costs since it must be ensured that the weldings are fluid tight irrespective of foil materials or amount of layers involved. Moreover, these known types of disposable urine bags include a relatively high amount of material, i.e. eight plastic layers in said upper section.

On this basis, it is an object by the present invention to provide a disposable fluid collection bag for hygienic purposes, in particular urine bag, of the initially mentioned kind, which is simple and inexpensive in manufacture.

This object is achieved by a fluid collection bag wherein the flexible non-return valve means extends into the fluid collection compartment, and that the valve means are mounted directly to the inlet opening collar.

By providing the flexible non-return valve means as a separate element, the non-return valve means may be made in a special flexible foil material, since the valve foils need not be welded together with the foils forming the pouch. By mounting the valve means directly to the inlet collar, the pouch is not divided into two compartments since the entire volume of the pouch may constitute the fluid collection compartment thereby increasing the capacity of the fluid collection bag.

This also means that when emptying the fluid collection bag, the bag may be punctured at its uppermost portion, above the fluid level and the fluid can be poured out through this opening. This is not possible by the hitherto known disposable urine bags due to the integrally formed non-return valve means.

According to the preferred embodiment, the non-return valve means comprises a plurality of foil layers of different lengths, where these foil layers extending from the inlet collar and are joined together to define a general flow direction into the fluid compartment. Moreover, discrete spot weldings are preferably provided at the end sections of each set of foil layers joining all the present the foil layers together. This means that the fluid will open the foils when flowing through in the direction from the inlet to the distal ends of the foils. However, if the a counter flow should occur, this will cause the flow passage between the foil layers to close, and thereby prevent the fluid from escaping the fluid compartment, once the fluid has passed the flexible foil arrangement of the non-return valve means.

The flexible pouch is preferably made of two flexible foil sheet layers of substantially identical shape joined together along their periphery with the inlet opening collar provided between said foil sheets, and that said sheets are joined to the inlet collar instead of each other at the position of the inlet opening collar of the bag. Hereby, the pouch is made and the inlet may be mounted and sealed in the same welding process.

In the preferred embodiment, the inlet opening collar is provided centrally on the pouch, which may be substantially rectangular in shape. By providing the inlet centrally on the upper side of the bag, the upper corner regions may be used for handling and emptying, which in turn provides for an easy handling of the bag during use.

The inlet collar is preferably moulded with a flattened configuration, which is openable by pushing at the two opposite folds in the flattened configuration against each other. Various inlet collar features may advantageously be provided.

A bag according to the invention may be provided with outlet means for emptying the fluid in the collection compartment. In a preferred embodiment, the outlet means is provided adjacent the inlet opening of the bag. Various outlet means may be provided.

In an advantageous embodiment of the invention, a test compartment is provided adjacent the outlet means in such a manner that the test compartment is filled with the fluid during the emptying of the pouch for collecting a sample e.g. for further analysis of the collected fluid.

A bag according to the invention may be made of flexible transparent or translucent plastic foil material.

By a urinal according to the invention, the advantageous differences include
- that the opening is placed in the middle of the upper part of the urinal and is provided with a spout, which opens and closes with a closure effect known from wallets, but not with two internal tongues, as in the former patent;
- that a tear corner is provided for easy disposal of the urine, this being placed in the upper part of the bag, which facilitates the emptying of the urinal as compared to a positioning in the lower part of the bag, and
- an inner sample pouch is provided near the tear corner for easy stick insertion through the torn corner for sampling the urine.

Apart from the above described embodiments, further embodiment of a collection bag for collecting bodily fluids, could be that the tear corner is provided with variable width tear ridges to facilitate a slow pouring when taking samples, or a fast pouring when disposing of the collected urine; and that lower part of the valve is provided with a urine test strip for easy recording of values, evading the problem of having urine poured over hands and the like during sampling.

In an embodiment of the invention, the inlet opening collar is an inlet tube. The tube may be connected to a catheter. In addition, the fluid compartment may be provided with a drainage tube with a stop valve.

The invention is described in more detail with reference to the accompanying drawings, in which:
- Fig. 1: shows a fluid collection bag according to a preferred embodiment of the invention;
- Fig. 2: shows the flexible foil arrangement in a non-return valve means;

- Fig. 3: is a perspective view of an inlet collar according to the invention;
- Fig. 4: shows the same from a bottom perspective view; and
- Figs. 5 to 7: are schematic views of three embodiments of a fluid collection bag according to the invention with outlet means.

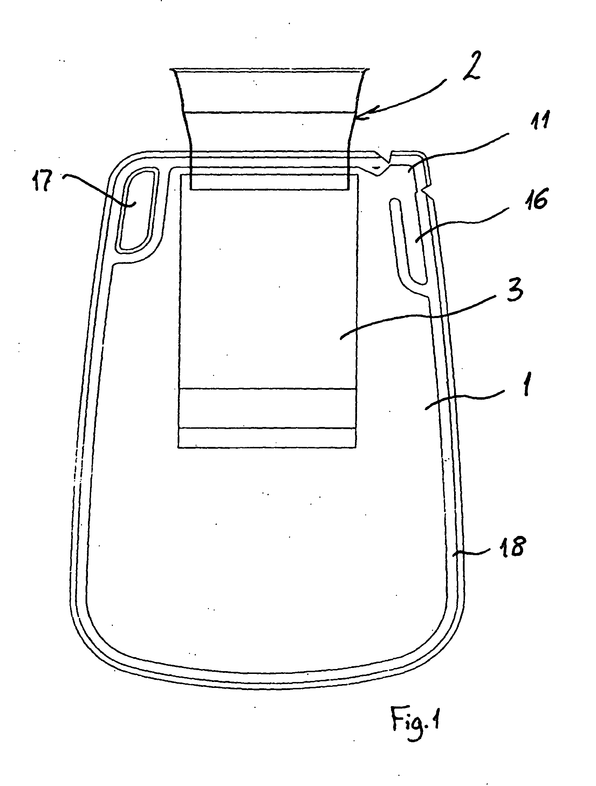

With reference to figure 1, a fluid collection bag according to a preferred embodiment of the invention is shown. The bag comprises a pouch 1, an inlet opening collar 2 and a flexible foil arrangement 3 forming the non-return valve means ensuring that fluid which flow through the inlet 2 and into the pouch 1 cannot escape through the foil arrangement 3. The foil arrangement 3 is mounted directly below the inlet collar 2 and extends into the pouch 1 as it is apparent in fig. 1.

In the pouch 1 there is formed a small compartment 16 and a tear strip 11 providing means for fluid outlet in the upper corner adjacent the centrally disposed inlet collar 2. In the opposite upper corner region of the pouch 1 a handling aperture 17 is provided for e.g. hanging the urine bag on a hook or the like and/or for holding the bag during filling and emptying fluids.

The pouch 1 includes two flexible foil layers, e.g. two identically shaped foil sheets as shown in fig. 1, or one foil which is folded. The foils are provided with a weld seam 18 along the periphery of the pouch 1. The non-return valve foil arrangement 3 is welded or otherwise permanently joined to the inlet collar 2. Subsequently, the inlet collar 2 is sandwiched between the foil layers making up the pouch and welded together with the foil layer to form the inlet of the pouch 1.

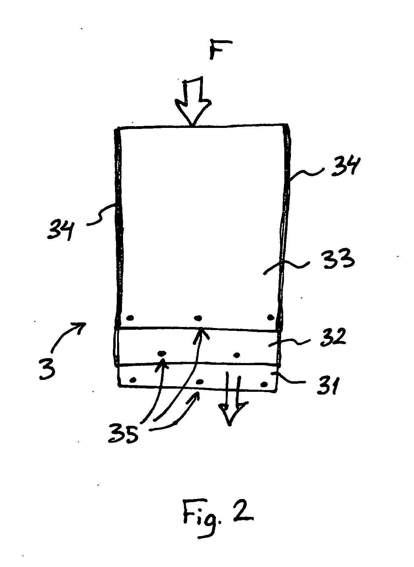

The non-return valve foil arrangement 3 is not welded to the sides of the pouch, as it is the case in the prior art, but extends from the inlet collar and into the fluid compartment of the pouch. The Non-return valve 3, which is shown in fig. 2, is made of six flexible foil layers formed in three pairs 31, 32, 33, where the foil layers 31, 32, 33 are welded along their sides 34 and a spot welded 35 at the distal end of the foil arrangement to form a directional flow path F through the non-return flexible foil valve arrangement 3. In this embodiment, the inner foil layers may be provided with a length of 10 cm, the pair of middle foil layers having a length of 13 cm and the outermost foil layers having a length of 14 cm. The material of the foil may be PELD, which is a low density polyethylene which is transparent and soft and highly flexible. However, it is realised that other types of flexible foil materials may be used, including both mono and multilayered foils. The inlet collar is made of a relatively rigid plastic material.

The point weldings 35 are provided in one or more lines, and the lowermost line could extend all the way across and also joining the outermost non-return valve foils 31 to the bag foils forming the pouch 1.

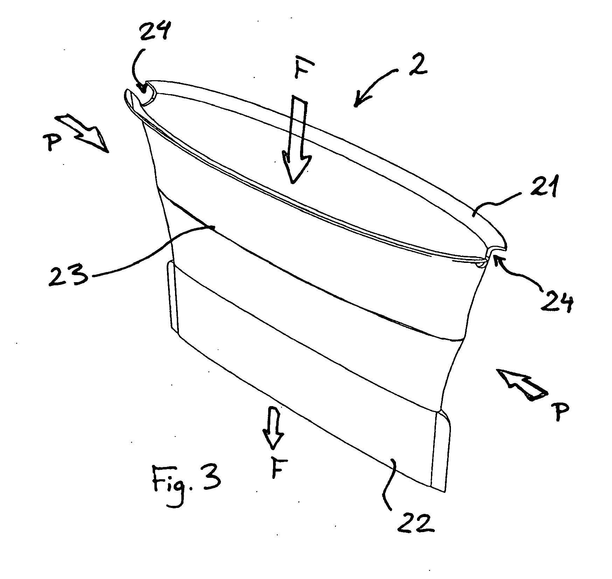

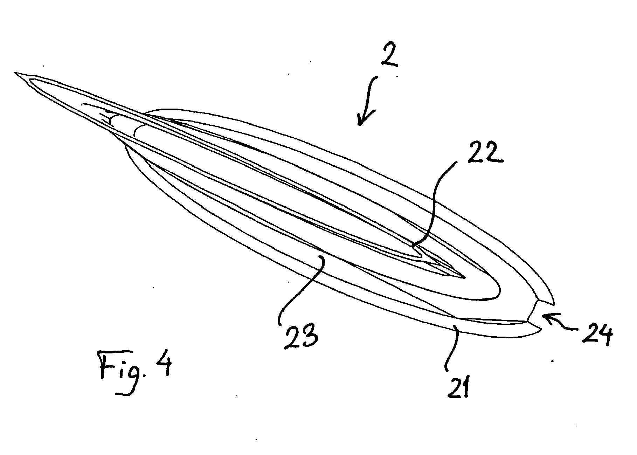

The inlet collar 2 is shown in more detail in figures 3 and 4. The inlet collar 2 is disposed in the central portion of the urine bag, as shown in fig. 1. The inlet collar 2 is made in a resilient plastic material and with a generally flat configuration, which is openable when pressure is applied to the side edges, as indicated by the arrows P. Hereby, the two side portions 23 bend outwards and creates the flow passage allowing the flow F to pass through the inlet opening. The outermost periphery 23 may be provided with a cut-out 24 at the two corner edges in order to avoid the inlet collar sides 23 to buckle when opening the inlet collar 2 by applying the inward pressure P on the folds.

As shown in fig. 3, the inlet opening collar 2 is preferably moulded with an elliptically shaped outermost end periphery 23 and a generally flat openable innermost end periphery 22.

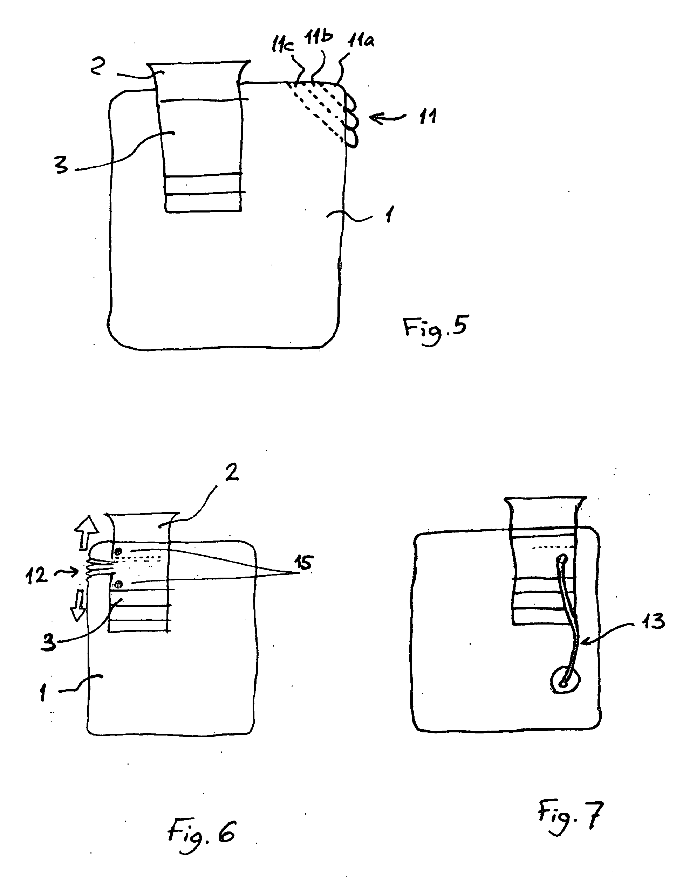

As described with reference to fig. 1, a tear strip 11 may be provided in the corner of the pouch for providing an outlet opening. As shown in fig. 5, another embodiment could be to provide a multiple of substantially congruently formed tear strips 11a, 11b, 11c in the corner region for providing a plurality of possible sizes of outlet opening.

Other embodiment of outlet means in a fluid collection bag according to the invention are shown in figures 6 and 7. For instance, as shown in fig. 6, the outlet means may include tearing means 12 for destroying the non-return valve 3 as two weld spots 15 are provided joining the flexible non-return valve 3 to the pouch foil. Another embodiment is shown in fig. 7, where a tearing strip 13 is provided in the pouch 1 for providing a by-pass of the non-return valve 3 and allowing the collected fluid to flow from the fluid collection compartment to the inlet opening 1.

The invention is described above with reference to some preferred embodiments. However, it is realised that other embodiments and variations of the fluid collection bag may be provided without departing from the scope of protection defined in the accompanying claims.

## Claims

1. A fluid collection bag for use for hygienic purposes, in particular a disposable urine bag, comprising:
a flexible foil pouch (1) defining a fluid collection compartment,
an inlet opening collar (2) mounted in the pouch (1) for allowing fluid being entered into the pouch (1), and
non-return valve means (3) comprising a plurality of flexible foil layers (31, 32, 33) of different lengths between the inlet opening collar (2) and the fluid collection compartment for preventing the fluid from being escaping the fluid collection compartment, said flexible non-return valve means (3) extending into the fluid collection compartment,
**characterised by** the valve means (3) being mounted directly to the inlet opening collar (2), where these foil layers (31, 32, 33) extend from the inlet collar and are joined together to define a general flow direction into the fluid compartment.

2. A fluid collection bag according to claim 1, wherein discrete spot weldings (35) are provided at the end sections of each set of foil layers (31, 32, 33) joining all the present the foil layers (31, 32, 33) together.

3. A fluid collection bag according to claim 1 or 2, wherein the flexible pouch (1) is made of two flexible foil sheet layers of substantially identical shape joined together along their periphery with the inlet opening collar (2) provided between said foil sheets, and that said sheets are joined to the inlet collar (2) instead of each other at the position of the inlet opening collar (2) of the bag.

4. A fluid collection bag according to any of the preceding claims wherein the pouch is substantially rectangular and wherein the inlet opening collar (2) is provided centrally on the substantially rectangular shaped pouch (1).

5. A fluid collection bag according to any of the preceding claims, wherein the inlet collar (2) is a tubular member formed with an openable, flattened configuration in a non-active position having two side portions (23) with two oppositely situated longitudinally oriented folds, and an exterior inlet periphery opening (21) and an internal periphery opening (22) and with a tapered collar between said two peripheral openings (21, 22), and that the tubular opening is established by applying an inwardly oriented pressure to the folds on the outermost periphery (21).

6. A fluid collection bag according to claim 5, wherein the inlet opening collar (2) comprises a set of collar members on the periphery of the inlet opening which in a flattened position closes the opening and which by applying pressure on the extreme ends of said collar members causes the members to bend and thereby provide an opening of the inlet.

7. A fluid collection bag according to claim 6, wherein the set of openable collar members is provided on the innermost periphery end (22) of the inlet collar.

8. A fluid collection bag according to any of claims 5 to 7, wherein the inlet opening collar (2) is provided with an elliptically shaped outermost end periphery (21) and a generally flat openable innermost end periphery (22).

9. A fluid collection bag according to any of claims 5 to 8, wherein the exterior inlet periphery opening (21) is provided with a cut-away (24) in connection to the folds for avoiding buckling of the collar (2) when activating the opening by applying an inward pressure on the folds.

10. A fluid collection bag according to any of the preceding claims, wherein the inlet opening collar (2) extends into the fluid compartment.

11. A fluid collection bag according to any of the preceding claims, wherein the inlet opening collar (2) extends out of the periphery of the pouch (1).

12. A fluid collection bag according to any of the preceding claims, wherein outlet means (11; 12, 15; 13) are provided for emptying the fluid in the collection compartment.

13. A fluid collection bag according to claim 12, wherein the outlet means (11; 12, 15; 13) is provided adjacent the inlet opening of the bag.

14. A fluid collection bag according to claim 12 or 13, wherein the outlet means comprises a tear strip (11), which is provided for providing an emptying opening, said tear strip (11) being provided in the corner of the pouch.

15. A fluid collection bag according to claim 14, wherein the outlet means comprises a multiple of substantially congruently formed tear strips (11a, 11b, 11c) are provided in a corner region for providing a plurality of possible sizes of the emptying opening.

16. A fluid collection bag according to claim 12, wherein the outlet means include a tearing strip (13) in the pouch for providing a by-pass of the non-return valve and allowing the collected fluid to flow from the fluid collection compartment to the inlet opening.

17. A fluid collection bag according to claim 12, wherein the outlet means include tearing means (12, 15) for destroying the non-return valve (3).

18. A fluid collection bag according to any of the preceding claims, wherein a test compartment (16) is provided adjacent the outlet means (11) in such a manner that the test compartment is filled with the fluid during the emptying of the pouch (1) for collecting a sample e.g. for further analysis of the collected fluid.

19. A fluid collection bag according to any of the preceding claims, wherein the bag is made of flexible transparent plastic foil material.

20. A fluid collection bag according to any of the preceding claims, wherein the bag is made of flexible translucent plastic foil material.

21. A fluid collection bag according to claim 1, wherein the inlet opening collar (2) is an inlet tube.

## Patentansprüche

1. Fluid-Sammelbeutel zur Verwendung zu hygienischen Zwecken, insbesondere ein Einweg-Urinbeutel, umfassend:
einen Beutel (1) aus flexibler Folie, der einen Fluid-Sammelraum definiert,
einen Einlaß-Öffnungskragen (2), der an dem Beutel (1) angebracht ist, damit Fluid in den Beutel (1) eingebracht werden kann, und
ein Rückschlagventil-Mittel (3), das mehrere flexible Folienlagen (31, 32, 33) unterschiedlicher Länge zwischen dem Einlaß-Öffnungskragen (2) und dem Fluid-Sammelraum umfasst, so dass das Fluid nicht aus dem Fluid-Sammelraum entweichen kann, worin sich das flexible Rückschlagventil-Mittel (3) in den Fluid-Sammelraum erstreckt, **dadurch gekennzeichnet, daß**
das Ventilmittel (3) direkt an dem Einlaß-Öffnungskragen (2) angebracht ist, worin sich die Folienlagen (31, 32, 33) von dem Einlaßkragen erstrecken und miteinander verbunden sind, um eine generelle Strömungsrichtung in den Fluid-Raum zu definieren.

2. Fluid-Sammelbeutel nach Anspruch 1, worin an den Endbereichen einer jeden Folienlage (31, 32, 33) separate Punktverschweißungen (35) bereitgestellt sind, die alle vorhandenen Folienlagen (31, 32, 33) zusammen verbinden.

3. Fluid-Sammelbeutel nach Anspruch 1 oder 2, worin der flexible Beutel (1) aus zwei flexiblen Folienlagen-Bahnen mit im Wesentlichen identischer Form hergestellt sind, die entlang ihres Umfangs verbunden sind, worin der Einlaß-Öffnungskragen (2) zwischen den Folienbahnen bereit gestellt ist, und worin die Bahnen anstelle miteinander an der Position des Einlaß-Öffnungskragens (2) des Beutels an den Einlaßkragen (2) verbunden sind.

4. Fluid-Sammelbeutel nach einem der vorhergehenden Ansprüche, worin der Beutel im wesentlichen rechteckig ist und worin der Einlaß-Öffnungskragen (2) zentral auf dem im Wesentlichen rechteckig ausgebildeten Beutel (1) bereitgestellt ist.

5. Fluid-Sammelbeutel nach einem der vorhergehenden Ansprüche, worin der Einlaßkragen (2) ein röhrenförmiges Element ist, das in einer nicht-aktiven Position mit einem zu öffnenden, abgeflachten Aufbau mit zwei Seitenbereichen (23) ausgebildet ist, die zwei gegenüberliegend angeordnete längs-orientierte Faltungen aufweisen, und einer äußeren Einlaß-Umfangsöffnung (21) und einer inneren Umfangsöffnung (22), und mit einem sich verjüngende Kragen zwischen den beiden Umfangsöffnungen (21, 22), und worin die röhrenförmige Öffnung durch Anlegen eines nach innen gerichteten Drucks an die Faltungen an dem äußersten Umfang (21) erstellt wird.

6. Fluid-Sammelbeutel nach Anspruch 5, worin der Einlaß-Öffnungskragen (2) am Umfang der Einlaßöffnung einen Satz Kragenelemente umfaßt, die in einer abgeflachten Position die Öffnung verschließen, und die an den äußersten Enden der Kragenelemente durch Ausüben von Druck die Elemente dazu bringen, sich zu biegen und **dadurch** eine Öffnung des Einlasses liefert.

7. Fluid-Sammelbeutel nach Anspruch 6, worin der Satz der zu öffnenden Kragenelemente an dem innersten Umfangsende (22) des Einlaßkragens bereitgestellt ist.

8. Fluid-Sammelbeutel nach einem der Ansprüche 5 bis 7, worin der Einlaßöffnungs-Kragen (2) mit einem elliptisch geformten äußersten Endumfang (21) und einem im Wesentlichen flachen, zu öffnenden Endumfang (22) bereitgestellt ist.

9. Fluid-Sammelbeutel nach einem der Ansprüche 5 bis 8, worin die äußere Einlaßumfangs-Öffnung (21) in Verbindung mit den Faltungen mit einem Schnitt (24) versehen ist, um ein Verziehen des Kragens (2) zu verhindern, wenn die Öffnung durch Anlegen eines inneren Drucks an die Faltungen aktiviert wird.

10. Fluid-Sammelbeutel nach einem der vorstehenden Ansprüche, worin sich der Einlaß-Öffnungskragen (2) in den Fluidraum erstreckt.

11. Fluid-Sammelbeutel nach einem der vorstehenden Ansprüche, worin sich der Einlaß-Öffnungskragen (2) aus dem Umfang des Beutels (1) erstreckt.

12. Fluid-Sammelbeutel nach einem der vorstehenden Ansprüche, worin Auslaßmittel (11, 12, 15, 13) zum Entleeren des Fluids in den Sammelraum bereitgestellt sind.

13. Fluid-Sammelbeutel nach Anspruch 12, worin Auslaßmittel (11, 12, 15, 13) neben der Einlaßöffnung des Beutels bereitgestellt sind.

14. Fluid-Sammelbeutel nach Anspruch 12 oder 13, worin das Auslaßmittel einen Aufreißstreifen (11) umfasst, der bereitgestellt ist, um eine Entleerungsöffnung bereitzustellen, wobei der Aufreißstreifen (11) in der Ecke des Beutels bereitgestellt ist.

15. Fluid-Sammelbeutel nach Anspruch 14, worin das Auslaßmittel mehrere im Wesentlichen gleich geformte Aufreißstreifen (11a, 11b, 11c) umfasst, die in einem Eckenbereich bereitgestellt wird, um mehrere mögliche Grössen der Entleerungsöffnungen bereitzustellen.

16. Fluid-Sammelbeutel nach Anspruch 12, worin das Auslaßmittel einen Aufreißstreifen (13) in dem Beutel umfasst, um eine Überbrückung des Rückschlagventils bereitzustellen, so daß das gesammelte Fluid von dem Fluid-Sammelraum zu der Einlaßöffnung strömen kann.

17. Fluid-Sammelbeutel nach Anspruch 12, worin das Auslaßmittel Reißmittel (12, 15) umfasst, um das Rückschlag-Ventil (3) zu zerstören.

18. Fluid-Sammelbeutel nach einem der vorstehenden Ansprüche, worin ein Testraum (16) neben dem Auslaßmittel (11) bereitgestellt wird, so dass der Testraum während des Entleerens des Beutels (1) zum Gewinnen einer Probe, beispielsweise zur weiteren Analyse des gesammelten Fluids, mit dem Fluid gefüllt wird.

19. Fluid-Sammelbeutel nach einem der vorstehenden Ansprüche, worin der Beutel aus einem flexiblen transparenten Kunststoff-Folienmaterial hergestellt ist.

20. Fluid-Sammelbeutel nach einem der vorstehenden Ansprüche, worin der Beutel aus einem flexiblen, durchsichtigen Kunststoff-Folienmaterial hergestellt ist.

21. Fluid-Sammelbeutel nach Anspruch 1, worin der Einlaßöffnungs-Kragen (2) eine Einläßröhre ist.

## Revendications

1. Sac de récupération de fluide à usage hygiénique, en particulier un sac à urine jetable, comprenant :
une poche en feuille souple (1) définissant un compartiment de récupération de fluide,
un collier d'ouverture d'entrée (2) monté sur la poche (1) pour permettre l'entrée du fluide dans la poche (1), et
un moyen de clapet anti-retour(3) comportant une pluralité de couches de feuille souple (31, 32, 33) de différentes longueurs entre le collier d'ouverture d'entrée (2) et le compartiment de récupération de fluide pour empêcher le fluide de s'échapper du compartiment de récupération de fluide, ledit moyen de clapet anti-retour souple (3) s'étendant dans le compartiment de récupération de fluide, **caractérisé en ce que**
le moyen de clapet anti-retour (3) étant monté directement sur le collier d'ouverture d'entrée (2), dans lequel ces couches de feuille (31, 32, 33) s'étendent du collier d'ouverture d'entrée et sont reliées entre elles pour définir une direction de flux générale dans le compartiment de fluide.

2. Sac de récupération de fluide selon la revendication 1, dans lequel des points de soudure discrets (35) sont prévus aux sections d'extrémité de chaque série de couches de feuille (31, 32, 33) reliant entre elles toutes les couches de feuilles actuelles (31, 32, 33).

3. Sac de récupération de fluide selon la revendication 1 ou 2, dans lequel la poche souple (1) est composée de deux couches de feuilles souples de forme sensiblement identique reliées les unes aux autres le long de leur périphérie avec le collier d'ouverture d'entrée (2) fourni entre lesdites feuilles et lesdites feuilles sont reliées au collier d'entrée (2) plutôt que chacune à la position du collier d'ouverture d'entrée (2) du sac.

4. Sac de récupération de fluide selon l'une quelconque des revendications précédentes dans lequel la poche est sensiblement rectangulaire et, dans lequel le collier d'ouverture d'entrée (2) est fourni centralement sur la poche de forme sensiblement rectangulaire (1).

5. Sac de récupération de fluide selon l'une quelconque des revendications précédentes, dans lequel le collier d'entrée (2) est un élément tubulaire formé avec une configuration aplatie, pouvant s'ouvrir dans une position non active ayant deux parties latérales (23) avec deux feuilles orientées longitudinalement placées l'une en face de l'autre, et une ouverture de périphérie d'entrée extérieure (21) et une ouverture de périphérie intérieure (22) et avec un collier conique entre les deux ouvertures périphériques (21, 22) et l'ouverture tubulaire est établie en appliquant une pression orientée vers l'intérieur aux feuilles sur la périphérie externe (21).

6. Sac de récupération de fluide selon la revendication 5, dans lequel le collier d'ouverture d'entrée (2) comprend un ensemble d'éléments de collier sur la périphérie de l'ouverture d'entrée qui dans une position aplatie ferme l'ouverture et qui, en appliquant la pression sur les terminaisons extrêmes desdits éléments de collier plie les éléments fournissant ainsi une ouverture de l'entrée.

7. Sac de récupération de fluide selon la revendication 6, dans lequel l'ensemble des éléments de collier pouvant s'ouvrir est prévu sur l'extrémité périphérique interne (22) du collier d'entrée.

8. Sac de récupération de fluide selon l'une quelconque des revendications 5 à 7, dans lequel le collier d'ouverture d'entrée (2) est doté d'une périphérie d'extrémité externe de forme elliptique (21) et une périphérie d'extrémité interne généralement plate pouvant s'ouvrir (22).

9. Sac de récupération de fluide selon l'une quelconque des revendications 5 à 8, dans lequel l'ouverture de périphérie d'entrée extérieure (21) est dotée d'une découpe (24) en relation avec les feuilles pour éviter la déformation du collier (2) lors de l'activation de l'ouverture par application d'une pression interne sur les feuilles.

10. Sac de récupération de fluide selon l'une quelconque des revendications précédentes, dans lequel le collier d'ouverture d'entrée (2) s'étend dans le compartiment de fluide.

11. Sac de récupération de fluide selon l'une quelconque des revendications précédentes, dans lequel le collier d'ouverture d'entrée (2) s'étend en dehors de la périphérie de la poche (1).

12. Sac de récupération de fluide selon l'une quelconque des revendications précédentes, dans lequel des moyens de sortie (11 ; 12 ; 15 ; 13) sont prévus pour vider le fluide dans le compartiment de récupération.

13. Sac de récupération de fluide selon la revendication 12, dans lequel les moyens de sortie (11 ; 12 ; 15 ; 13) sont prévus à proximité de l'ouverture d'entrée du sac.

14. Sac de récupération de fluide selon la revendication 12 ou 13, dans lequel le moyen de sortie comprend une bande détachable (11) qui est prévue en tant qu'ouverture de vidage, ladite bande détachable (11) étant prévue dans le coin de la poche.

15. Sac de récupération de fluide selon la revendication 14, dans lequel le moyen de sortie comprend plusieurs bandes détachables formées de façon sensiblement congruente (11a, 11b, 11c) prévues dans une région de coin pour fournir une pluralité de dimensions possibles d'ouverture de vidage.

16. Sac de récupération de fluide selon la revendication 12, dans lequel le moyen de sortie comprend une bande détachable (13) dans la poche pour fournir une dérivation du clapet anti-retour et permettre au fluide récupéré de s'écouler du compartiment de récupération de fluide vers l'ouverture d'entrée.

17. Sac de récupération de fluide selon la revendication 12, dans lequel le moyen de sortie comprend des moyens détachables (12, 15) pour détruire le clapet anti-retour (3).

18. Sac de récupération de fluide selon l'une quelconque des revendications précédentes, dans lequel un compartiment d'essai (16) est prévu à proximité du moyen de sortie (11) de sorte que le compartiment d'essai soit rempli avec le fluide au cours du vidage de la poche (1) afin de récupérer un échantillon par exemple pour une analyse ultérieure du fluide récupéré.

19. Sac de récupération de fluide selon l'une quelconque des revendications précédentes, dans lequel le sac est composé d'un matériau de feuille plastique transparent souple.

20. Sac de récupération de fluide selon l'une quelconque des revendications précédentes, dans lequel le sac est composé d'un matériau de feuille plastique translucide souple.

21. Sac de récupération de fluide selon la revendication 1, dans lequel le collier d'ouverture d'entrée (2) est un tube d'entrée.
